# EUROPEAN PATENT APPLICATION

(11) **EP 3 252 147 A1**
(43) Date of publication of application: **06.12.2017**
(21) Application number: 16742904.2
(22) Date of filing: 04.01.2016
(51) Int. Cl.: C12N 5/07, C12M 1/00, C12M 3/02

(54) **METHOD OF TRANSFERRING LIQUID BETWEEN MULTIPLE VESSELS IN CELL CULTIVATION**

(30) Priority: 30.01.2015 JP 2015017826
(71) Applicant: Toyo Seikan Group Holdings, Ltd., Shinagawa-ku Tokyo 141-8627 (JP)
(72) Inventor: HATA, Norihiko, Tokyo 141-8627 (JP); MURAI, Masahiro, Tokyo 141-8627 (JP)
(74) Representative: Liebetanz, Michael
(86) International application number: PCT/JP2016/000006
(87) International publication number: WO 2016/121295

(57) **Abstract**

Enable to simplify control of a flow path between containers when cell culture is conducted by using multiple containers. A first culture container of multiple culture containers each having a port for transferring a content, a three-way stopcock and a culture medium supply container having one port are connected by a tube, and one pump is arranged between the three-way stopcock and the culture medium supply container; when the number of culture containers in the multiple culture containers is two, the three-way stopcock and a second culture container in the multiple culture containers are connected by a tube; when the number of culture containers in the multiple culture containers is three or more, a three-way stopcock is newly provided between the culture container immediately connected and the three-way stopcock, and connection of the newly provided three-way stopcock and a subsequent culture container by a tube is repeated, whereby all culture containers are connected to the three-way stopcock; and the culture medium is transferred from the culture medium supply container to each culture container in the multiple culture containers via the three-way stopcock by the pump.

## Description

### Technical Field

The present invention relates to a cell culture technology. In particular, the present invention relates to a method of transferring a liquid between containers when multiple culture containers and a culture medium supply container are used.

### Background Art

In recent years, in the field of production of medicine, gene therapy, regenerative therapy, immunotherapy etc., it is required to culture efficiently a large amount of cells, tissues, microorganisms, etc. in an artificial environment. In such cell culture, there may be a case where a method is adopted in which multiple containers are connected by a tube to create a closed environment, and liquid transfer is conducted between these multiple containers.

Specifically, for example, the following method, etc. are conducted: multiple culture bags and a culture medium supply bag are connected by a tube, a culture medium is transferred from the culture medium supply bag to the culture bags, cells are cultured in the culture bags, a culture solution containing the cultured cells and the culture medium (hereinafter, one containing cells and a culture medium is referred to as a "culture solution") is transferred to a culture bag having a larger culture area, thereby to further proliferate the cells.

At this time, in order to transfer a content between the multiple bags, it is possible to transfer the content efficiently if a pump is provided on between every bags. However, this method has a problem that not only the cost is increased, but also a complicated control mechanism and many devices become necessary.

On the other hand, it is possible to reduce the number of pumps by branching a tube. However, in this case, a problem arises that a pinch valve or the like may be required in order to control the flow path of the branched tube, thus leading to the need of a complicated control mechanism and many devices.

Here, Patent Document 1 discloses a container kit for cell culture for use in an automatic cell culture device. According to this kit for cell culture, a process in cell culture including injection of cells, addition of a culture medium, sampling and collection can be conducted while maintaining a closed system.

Patent Document 1: WO2013/114845

### Summary of the Invention

### Problems to be Solved by the Invention

However, this kit for cell culture was not contrived in order to simplify the control mechanism of a flow path in cell culture. Therefore, this kit involves problems that not only one or plural pumps are used, but also a large number of clips are required to be used when a flow path between the multiple containers is controlled, thereby making the control mechanism of a flow path slightly complicated.

The inventors of the present invention made intensive studies. As a result, they succeeded in using only one pump and easy control of a flow path by connecting a plurality of containers through a three-way stopcock and by arranging culture containers so that heights are difference in the order of liquid transfer. The present invention has been thus completed.

According to the present invention, transfer of a culture medium from a culture medium supply container to a culture container can be conducted by a pump, and transfer of a culture solution between multiple culture containers can be conducted by natural liquid transfer. Further, use of a pinch valve, a clip or the like becomes unnecessary, whereby control mechanism can be greatly simplified.

The present invention has been made taking the above circumstances into consideration, and is aimed at providing a method of transferring a liquid between multiple culture containers in cell culture, which method is capable of simplifying control of a flow path between the containers when cell culture is conducted by using multiple containers.

### Means for Solving the Problem

In order to attain the object, the method of transferring a liquid between multiple culture containers in cell culture according to the present invention is a method of transferring a liquid between multiple containers in cell culture in which a culture medium supply container having one port and multiple culture containers each having one port for transferring a content through which a culture medium is supplied from the culture medium supply container are used, wherein
a first culture container in the multiple culture containers, a three-way stopcock and the culture medium supply container are connected by a tube, and one pump is arranged between the three-way stopcock and the culture medium supply container,
when the number of culture containers in the multiple containers is two, the three-way stopcock and a second culture container in the multiple culture containers are connected by a tube,
when the number of culture containers in the multiple containers is three or more, newly provision of a three-way stopcock on the tube between the immediately connected culture container and the three-way stopcock, and connection of the newly provided three-way stopcock and a subsequent culture container by a tube are conducted repeatedly, whereby all culture containers are connected to the three-way stopcock, and
the culture medium is transferred from the culture medium supply container to each culture container in the multiple culture containers via the three-way stopcock by the pump.

### Advantageous Effects of the Invention

According to the present invention, it is possible to simplify the control of a flow path between containers when cell culture is conducted by using multiple containers.

### Brief Description of the Drawings

Fig. 1 is a front view showing the connection relationship of containers used in the method of transferring a liquid between multiple containers in cell culture according to a first embodiment of the present invention;
Fig. 2 is a plan view showing the connection relationship of containers used in the method of transferring a liquid between multiple containers in cell culture according to the first embodiment of the present invention;
Fig. 3 is a front view showing the connection relationship of containers used in the method of transferring a liquid between multiple containers in cell culture according to a second embodiment of the present invention; and
Fig. 4 is a front view showing the connection relationship of containers used in the method of transferring a liquid between multiple containers in cell culture according to a third embodiment of the present invention.

### Mode for Carrying out the Invention

Hereinbelow, preferable embodiments of the method of transferring a liquid between multiple containers in cell culture according to the present invention will be explained with reference to the drawings.

First, the characteristic features of the method of transferring a liquid between multiple containers in cell culture according to the embodiment of the present invention will be roughly explained.

The method of transferring a liquid between multiple culture containers in cell culture according to the embodiment of the present invention is a method of transferring a liquid between multiple containers in cell culture in which a culture medium supply container having one port and multiple culture containers each having one port for transferring a content through which a culture medium is supplied from the culture medium supply container are used, wherein a first culture container in the multiple culture containers, a three-way stopcock and the culture medium supply container are connected by a tube, and one pump is arranged between the three-way stopcock and the culture medium supply container, when the number of culture containers in the multiple containers is two, the three-way stopcock and a second culture container in the multiple culture containers are connected by a tube, when the number of culture containers in the multiple containers is three or more, newly provision of a three-way stopcock on the tube between the immediately connected culture container and the three-way stopcock, and connection of the newly provided three-way stopcock and a subsequent culture container by a tube are conducted repeatedly, whereby all culture containers are connected to the three-way stopcock, and the culture medium is transferred from the culture medium supply container to each culture container in the multiple culture containers via the three-way stopcock by the pump.

Also, it is preferred that the method of transferring a liquid between the multiple containers in cell culture according to the embodiment of the present invention have a configuration in which each culture container in the multiple culture containers is arranged such that the horizontal position of the previously connected culture container becomes higher than the horizontal position of the subsequently connected culture container, and transfer of a liquid from the previously connected culture container to the subsequently connected culture container is conducted by natural liquid transfer based on difference in horizontal position.

Further, it is preferred that the method of transferring a liquid between the multiple containers in cell culture according to the embodiment of the present invention have a configuration in which a supernatant of a culture solution or a culture solution is transferred from the last one of the subsequently connected culture containers to the culture medium supply container via the three-way stopcock by a pump.

Furthermore, it is preferred that the method of transferring a liquid between the multiple containers in cell culture according to the embodiment of the present invention have a configuration in which a three-way stopcock is provided on the tube between the culture medium supply container and the pump, one or two or more another culture medium supply containers each having one port are connected to the culture medium supply container via a three-way stopcock by a tube, and the culture medium is transferred from these culture medium supply containers to each culture container in the multiple culture containers via a three-way stopcock by the pump.

According to the method of transferring a liquid between multiple containers in cell culture according to the embodiment of the present invention, it becomes possible to adequately control a flow path by using only one pump in a closed cell culture system.

That is, by this method, multiple containers are connected by a three-way stopcock and one pump, and culture containers are arranged with differences in height being provided between the culture containers in the order of liquid transfer.

Therefore, transfer of a culture medium from a culture medium supply container to a culture container can be conducted by a pump, and transfer of a culture solution between multiple culture containers can be conducted by natural liquid transfer based on difference in horizontal position of culture containers. Further, by this method, since there is no need to use a pinch valve, a clip or the like, it becomes possible to greatly simplify the control mechanism of a flow path that was conventionally complicated.

### (First embodiment)

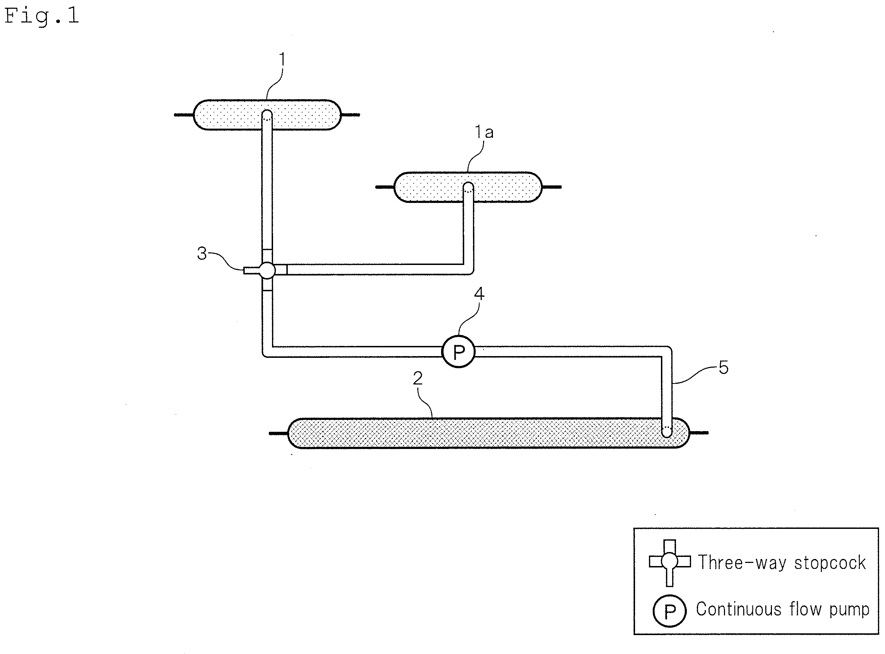

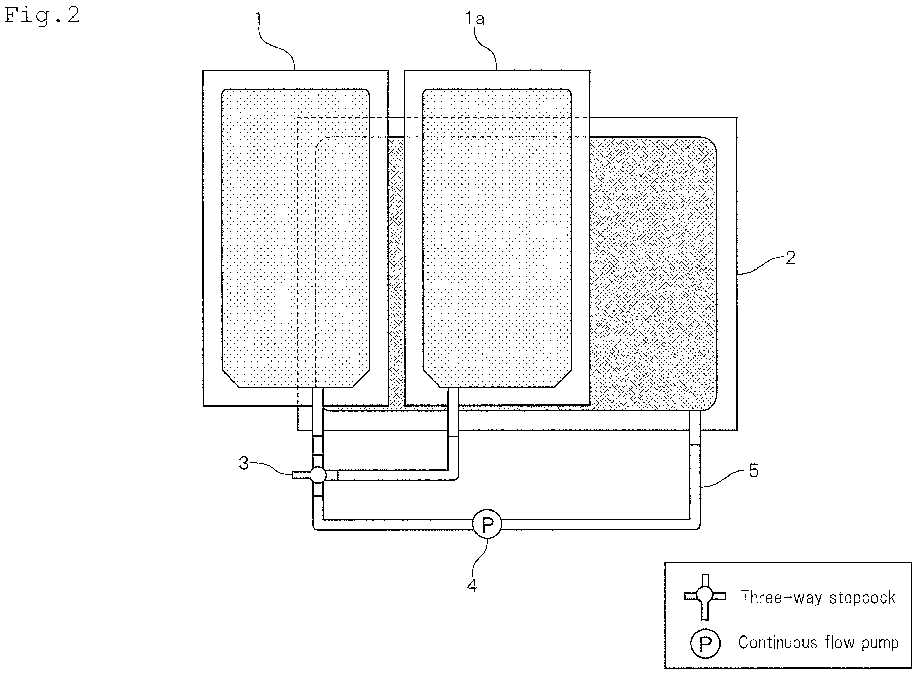

Subsequently, the method of transferring a liquid between multiple containers in cell culture according to a first embodiment of the present invention will be explained with reference to Fig. 1 and Fig. 2. Fig. 1 is a front view showing the connection relationship of containers used in the method of transferring a liquid between the multiple containers in cell culture according to the embodiment of the present invention, and Fig. 2 is a plan view thereof.

The cell culture system used in the present embodiment is provided with two culture containers.

That is, as shown in Fig. 1 and Fig. 2, the cell culture system used in the embodiment of the present invention is provided with a culture container 1, a culture container 1a, a culture medium supply container 2, a three-way stopcock 3, a pump 4 and a tube 5 connecting these.

The culture container 1, the culture container 1a and the culture medium supply container 2 are connected by the tube 5 via the three-way stopcock 3. In each container, one port for transferring a content with other containers is provided, and the tube 5 is connected with this port.

To the tube 5 between the three-way stopcock 3 and the culture medium supply container 2, the pump 4 is provided.

Further, the containers are arranged such that the horizontal position of the culture container 1 (which is previously connected) for conducting culture at first is higher than the horizontal position of the culture container 1a (which is subsequently connected) for conducting culture subsequently.

When using such a cell culture system, a culture solution containing cells and culture medium is injected into the culture container 1 under a sterile environment, and the culture container 1, the culture container 1a, the culture medium supply container 2 and the three-way stopcock 3 are connected by the tube 5, thereby to form a closed system.

Further, the pump 4 is provided and arranged between the culture medium supply container 2 and the three-way stopcock 3. Furthermore, the containers are arranged such that the horizontal position of the culture container 1 becomes higher than the horizontal position of the culture container 1 a. Then, via the three-way stopcock 3, a culture medium is transferred to the culture container 1 from the culture medium supply container 2 by the pump 4, whereby cell culture is conducted in the culture container 1.

When cell culture in the culture container 1 is completed, the culture solution is transferred from the culture container 1 to the culture container 1a by natural liquid transfer, and the culture medium is transferred from the culture medium supply container 2 to the culture container 1 a by the pump 4 via the three-way stopcock 3 and cell culture is conducted in the culture container 1a.

At this time, it is preferred that the culture solution in the culture container be stirred prior to the natural liquid transfer. When conducting natural liquid transfer, it is preferred that a mounting table on which the culture container is mounted be inclined such that a port provided on the culture container be directed downward. By this configuration, it is possible to conduct natural liquid transfer between the culture containers efficiently. Such technique of natural liquid transfer can be conducted in the same way as in the following embodiments.

Further, in this case, in order to warm the culture medium, at first, the culture medium is transferred from the culture medium supply container 2 to the culture container 1 by the pump 4 through the three-way stopcock 3. After the culture medium is sufficiently warmed in the culture container 1, the culture medium can be transferred by natural liquid transfer to the culture container 1a. Such warming of the culture medium can be conducted in the same way as in each of the culture containers in the following embodiments.

When the cell culture in the culture container 1 a is completed, a supernatant of the culture solution is transferred via the tube 5 and the three-way stopcock 3 by the pump 4 from the culture container 1 a to the culture medium supply container 2 that is empty, whereby the culture solution in the culture container 1 a can be concentrated. Similarly, the culture solution can be transferred from the culture container 1a to the culture medium supply container 2.

It is preferred that the capacity and the culture area of the culture container be designed such that the capacity and the culture area of a subsequently connected culture container be larger so as to allow a subsequently connected culture container can culture a large amount of cells.

It is preferred that the culture container 1 and the culture container 1a be formed of a soft packaging material and be formed into a bag-like shape. Further, in order to confirm a content, it is preferred that part or all thereof have transparency.

Further, these culture containers are required to have permeability to gases (permeability to oxygen and carbon dioxide) required for cell culture. It is preferable to use the container under culture conditions of 37°C and 5% carbon dioxide concentration. Furthermore, in order to realize a high cell proliferation efficiency, it is preferred that these culture containers have low cytotoxicity, low elution properties, and suitability to radiation sterilization.

As the material of the culture containers that satisfies such conditions, a polyethylene-based resin is preferable. As the polyethylene-based resin, polyethylene, a copolymer of ethylene and α-olefin, a copolymer of ethylene and vinyl acetate, an ionomer obtained by using a copolymer of ethylene and acrylic acid or methacrylic acid and a metal ion, or the like can be given. Further, polyolefin, a styrene-based elastomer, a polyester-based thermoplastic elastomer, a silicone-based thermoplastic elastomer, a silicone resin or the like can also be used.

The four sides of the culture container can be sealed by heat sealing. The shape of the accommodation part of the culture container 1 may be rectangular. Alternatively, as shown in Fig. 2, it is preferred that the culture container be configured such that it becomes gradually narrow towards the port. The culture container may be an integrally-molded bag obtained by blow molding.

It is preferred that the culture medium supply container 2 have gas barrier properties against oxygen and carbon dioxide in order to prevent pH of the culture medium stored from being changed largely during culture. The reason therefor is that, in order to prevent a culture medium from increasing pH due to escaping carbon dioxide contained in a culture medium at a high concentration to the air and decreasing the carbon dioxide concentration in the culture medium, it is desired that leakage of carbon dioxide from the inside of the culture medium supply container 2 to the outside be suppressed as much as possible. Further, it is desirable to prevent oxidation of a culture medium.

The type of the three-way stopcock 3 is not particularly restricted. However, for easiness in realization of a closed system in the cell culture system, a closed three-way stopcock can preferably be used.

The type of the pump 4 is not particularly restricted. However, in the similar viewpoint, a tube roller pump such as a Perista Pump (registered trademark) can preferably be used.

The material of the tube 5 may be appropriately selected according to the use environment. When used within a CO₂ incubator, it is desirable to use one having excellent gas permeability for oxygen and carbon dioxide. When used outside a CO₂ incubator, the material of the tube is preferably one having excellent gas barrier properties. For example, silicone rubber, soft vinyl chloride resins, polybutadiene resins, ethylene-vinyl acetate copolymers, chlorinated polyethylene resins, polyurethane-based thermoplastic elastomers, polyester-based thermoplastic elastomers, silicone-based thermoplastic elastomers, styrene-based elastomers or the like can be used. As the styrene-based elastomer, for example, SBS (styrenebutadiene-styrene), SIS (styrene-isoprene-styrene), SEBS (styrene-ethylenebutylene-styrene), SEPS (styrene-ethylene-propylene-styrene) or the like can be used.

In particular, in a part of the tube 5 at which a pump is provided, a silicone tube can preferably be used. In other parts, a soft vinyl chloride resin tube can be preferably used.

The type of the cultured cells in this embodiment is not particularly restricted. For example, floating cells such as lymphocytes, adherent cells such as skin cells, corneal cells, vascular endothelial cells, and mesenchymal stem cells can be preferably cultured.

### (Second embodiment)

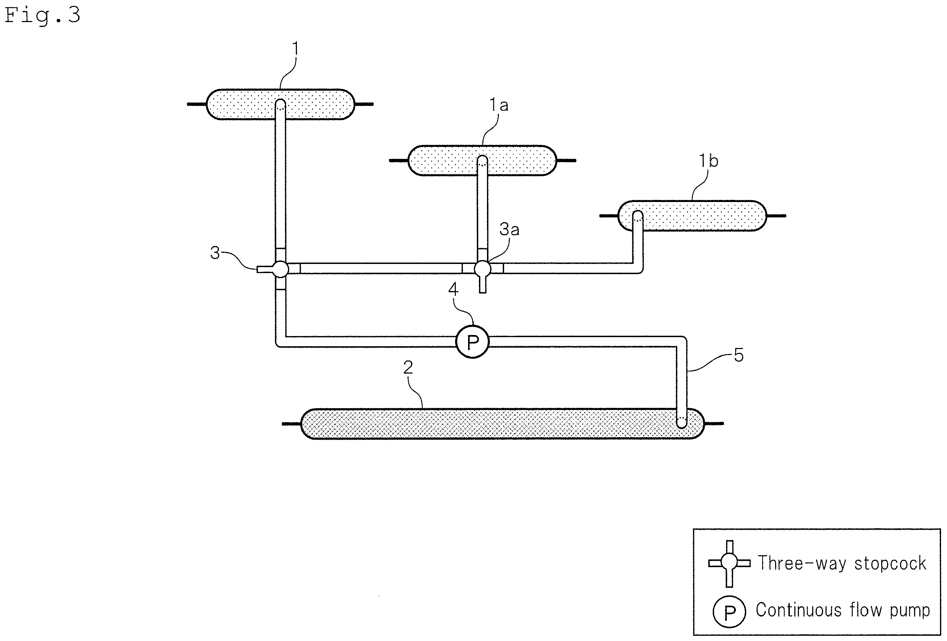

Subsequently, the method of transferring a liquid between multiple containers in cell culture according to a second embodiment of the present invention will be explained with reference to Fig. 3. Fig. 3 is a front view showing the connection relationship of containers used in the method of transferring a liquid between the multiple containers in cell culture according to the present embodiment.

The cell culture system used in the present embodiment is provided with three culture containers.

That is, as shown in Fig. 3, the cell culture system used in the present embodiment is provided with a culture container 1, a culture container 1 a, a culture container 1 b, a culture medium supply container 2, a three-way stopcock 3, a three-way stopcock 3a, a pump 4, and a tube 5 connecting these.

As in the case of the first embodiment, the culture container 1, the culture container 1 a and the culture medium supply container 2 are connected by the tube 5 via the three-way stopcock 3. Further, the three-way stopcock 3 and the three-way stopcock 3a are connected by the tube 5, and the culture container 1 a, the three-way stopcock 3a and the culture container 1 b are connected by the tube 5. In each container, one port for transferring a content with other containers is provided, and the tube 5 is connected to this port.

To the tube 5 between the three-way stopcock 3 and the culture medium supply container 2, the pump 4 is provided.

Further, the containers are arranged such that the horizontal position of the culture container 1 (which is previously connected) for conducting culture at first becomes higher than the horizontal position of the culture container 1 a (which is subsequently connected) for conducting culture subsequently, and the horizontal position of the culture container 1a becomes higher than the horizontal position of the culture container 1 b (which is further subsequently connected) for conducting culture further subsequently.

In the present embodiment, a configuration may be possible in which four or more culture containers are provided, and in the same way as in the culture container 1 a and the culture container 1 b, they are connected to the culture container 1 and the culture medium supply container 2 via the three-way stopcock by the tube 5. In this case, as in the case of the culture container 1 a and the culture container 1 b, it is preferred that the containers be arranged such that the horizontal position of the previously connected culture container is higher than the horizontal position of the subsequently connected culture container, whereby a cell suspension can be naturally transferred from the previously connected culture container to the subsequently connected culture container.

Further, in a culture container in which culture is conducted lastly, it is preferred that a port for sampling is separately provided, and a sampling tube is connected to this port.

When using such a cell culture system, a culture solution containing cells and a culture medium is injected into the culture container 1 under a sterile environment, and the culture container 1, the culture container 1 a, the culture container 1 b, the culture medium supply container 2, the three-way stopcock 3 and the three-way stopcock 3a are connected by using the tube 5, thereby to form a closed system.

Further, between the culture medium supply container 2 and the three-way stopcock 3, the pump 4 is provided. Furthermore, the containers are arranged such that the horizontal position of the culture container 1 becomes higher than the horizontal position of the culture container 1 a and the horizontal position of the culture container 1 a become higher than the horizontal position of the culture container 1 b. Then, via the three-way stopcock 3, the culture medium is transferred to the culture container 1 from the culture medium supply container 2 by the pump 4, whereby cell culture is conducted in the culture container 1.

When cell culture in the culture container 1 is completed, the culture solution is transferred from the culture container 1 to the culture container 1a by natural liquid transfer, and the culture medium is transferred from the culture medium supply container 2 to the culture container 1 a by the pump 4 via the three-way stopcocks 3 and 3b, and cell culture is conducted in the culture container 1a.

Further, when cell culture in the culture container 1a is completed, subsequently, the culture solution is transferred from the culture container 1a to the culture container 1 b by natural liquid transfer, and the culture medium is transferred from the culture medium supply container 2 to the culture container 1 b by the pump 4 via the three-way stopcocks 3 and 3a, whereby cell culture is conducted in the culture container 1 b.

When the cell culture in the culture container 1 b is completed, a supernatant of the culture solution is transferred via the three-way stopcock 3a and the three-way stopcock 3 by the pump 4 from the culture container 1 b to the culture medium supply container 2 that is empty, whereby the culture solution in the culture container 1 b can be concentrated. Similarly, the culture solution can be transferred from the culture container 1 b to the culture medium supply container 2.

In the present embodiment, when four or more culture containers are provided, as in the same manner mentioned above, the culture solution is transferred by natural liquid transfer from the previously connected culture containers to the subsequently connected culture container, and cell culture is conducted in the subsequently connected culture container. When the cell culture in the culture container lastly connected is completed, a supernatant of the culture solution is transferred via plural three-way stopcocks by the pump 4 from this culture container to the culture medium supply container 2 that is empty, whereby the culture solution in this culture container can be concentrated. Similarly, the culture solution can be transferred from the culture container to the culture medium supply container 2.

The type of culture cells in this embodiment is not particularly restricted, and floating cells such as lymphocytes can be preferably cultured.

In this case, the culture container 1 can preferably be used as a container for conducting curing culture and expansion culture for recovering the functions of damaged cells. That is, cells of which the functions have been lowered by being damaged (e.g. cells immediately after being taken out from a patient or cells that have been thawed after frozen storage) cannot be fully activated as they are. Therefore, such damaged cells cannot be cultured in a large amount. In order to proliferate these cells, a culture step in which the cells are cultured while recovering them in the initial stage of cell culture (curing culture step) is conducted so that the functions inherent to cells are recovered. Then, after the functions inherent to cells are recovered and proliferation of cells proceeds to some extent, the culture area is enlarged, and a culture step in which cell culture is continued until a prescribed cell density is attained (expansion culture step) is conducted. Therefore, in this case, it is preferred that the culture container 1 have an expandable culture area, and the means therefor is not particularly restricted.

Further, in this case, it is preferred that the culture container 1 a be used as an activation culture container used in a culture step for activating cells (activation culture step). On a bottom surface of the culture container 1 a, a substance such as anti-CD3 antibodies that activates cells is immobilized, and cells accommodated within the culture container 1 a are activated by binding to the substance. The anti-CD3 antibodies are preferably used in order to activate lymphocytes.

Furthermore, in this case, the culture container 1 b is preferably used as an amplification culture container used in a culture step to proliferate a large amount of cells (amplification culture step).

It is preferred that the capacity of the culture container 1 a be larger than the capacity of the culture container 1. For example, one having twice as large as the capacity of the culture container 1 can be preferably used. Since the culture container 1 b is used in order to proliferate a large amount of cells, it is preferable to use one having a particularly large capacity. For example, one having a capacity about ten times as large as the capacity of the culture container 1a can be preferably used.

In this embodiment, adherent cells such as skin cells, corneal cells, vascular endothelial cells, and mesenchymal stem cells can be cultured. In this case, for example, a configuration is preferable in which a three-way stopcock is provided in the tube 5 between the culture medium supply container 2 and the pump 4, and an enzyme solution supply container in which an enzyme solution for detaching cells from the inner surface of the culture container is enclosed and an inhibitor solution supply container in which an inhibitor solution for inhibiting the enzyme from working is enclosed are connected via the three-way stopcock. Further, it is possible to connect similarly other containers such as a washing liquid supply container and a waste solution collection container.

In this case, as for the capacity and the culture area of the culture container, it is preferred that a subsequently connected container have a larger capacity and a larger culture area in order to it possible to conduct efficiently subculture and so on.

In this embodiment, as for the material, properties, shape or the like of the culture container, they may be the same as those of the first embodiment. Further, as for the properties of the culture medium supply container 2, the type of the three-way stopcock 3 and the pump 4 and the material of the tube 5, they may be the same as those of the first embodiment.

### (Third embodiment)

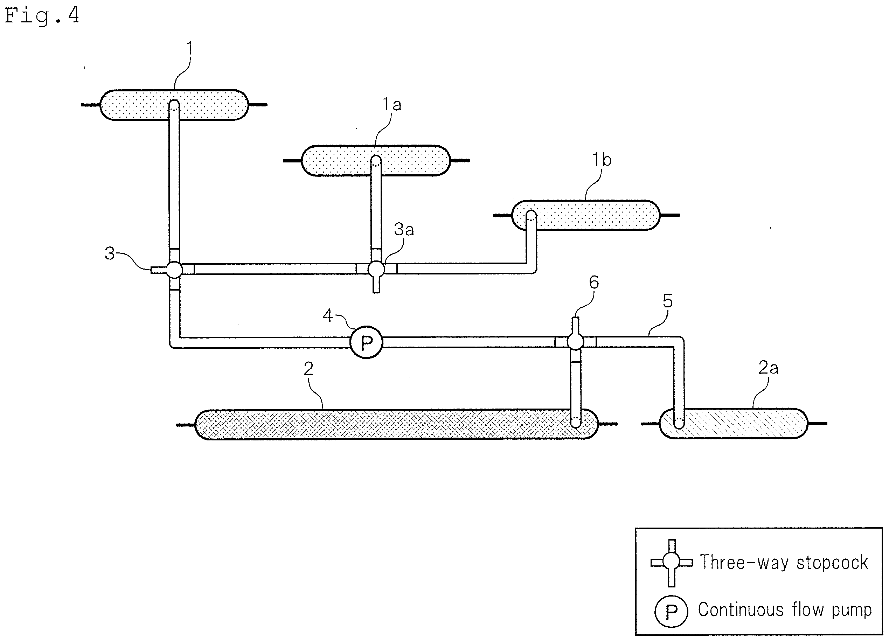

Subsequently, the method of transferring a liquid between multiple containers in cell culture according to a third embodiment of the present invention will be explained with reference to Fig. 4. Fig. 4 is a front view showing the connection relationship of containers used in the method of transferring a liquid between multiple containers in cell culture according to the present embodiment.

The cell culture system used in the present embodiment is provided with three culture containers, and two culture medium supply containers. Other points are the same as those in the second embodiment.

That is, as shown in Fig. 4, the cell culture system used in the present embodiment is further provided with a three-way stopcock 6 and a culture medium supply container 2a in addition to the configuration of the second embodiment, and the three-way stopcock 3 and the three-way stopcock 6 are connected by the tube 5, and the culture medium supply container 2, the three-way stopcock 6 and the culture medium supply container 2a are connected by the tube 5.

The properties of these culture medium supply containers may be the same as those of the first embodiment. In the culture medium supply container 2 and the culture medium supply container 2a, the same culture medium may be enclosed and different culture media may be enclosed.

In the present embodiment, a configuration may be possible in which three or more culture medium supply containers are provided, and in the same way as in the culture medium supply container 2a, they are connected to the culture medium supply container 2 and each culture container by the tube 5 via the three-way stopcock.

This embodiment mentioned above can be particularly preferably used when different culture media are supplied to multiple culture containers.

For example, a culture medium is supplied from the culture medium supply container 2a to the culture container 1 and the culture container 1a, the above-mentioned curing culture step and the expansion culture step are conducted in the culture container 1, and the above-mentioned activation culture step can be conducted in the culture container 1a. Further, subsequently, a culture medium is supplied from the culture medium supply container 2 to the culture container 1 b, and the above-mentioned amplification culture step can be conducted in the culture container 1 b.

Hereinabove, the present invention is explained with reference to preferable embodiments. It is needless to say the present invention is not restricted to the above-mentioned embodiments, and various modifications are possible within the scope of the present invention.

Appropriate modifications are possible; e.g. in the first embodiment, multiple culture medium supply containers may be provided as in the third embodiment, and other containers may be additionally provided.

### Industrial Applicability

The present invention can be preferably used in gene therapy, immunotherapy, regenerative therapy, production of antibody medicine in which a large amount of cells is cultured in a closed system.

The documents described in the specification and the specification of Japanese application(s) on the basis of which the present application claims Paris convention priority are incorporated herein by reference in its entirety.

### Explanation of Numerical Symbols

1, 1 a, 1 b Culture container
2, 2a Culture medium supply container
3, 3a, 3b Three-way stopcock
4 Pump
5 Tube
6 Three-way stopcock

## Claims

1. A method of transferring a liquid between multiple containers in cell culture in which a culture medium supply container having one port and multiple culture containers each having one port for transferring a content through which a culture medium is supplied from the culture medium supply container are used, wherein
a first culture container in the multiple culture containers, a three-way stopcock and the culture medium supply container are connected by a tube, and one pump is arranged between the three-way stopcock and the culture medium supply container,
when the number of culture containers in the multiple containers is two, the three-way stopcock and a second culture container in the multiple culture containers are connected by a tube,
when the number of culture containers in the multiple containers is three or more, newly provision of a three-way stopcock on the tube between the immediately connected culture container and the three-way stopcock, and connection of the newly provided three-way stopcock and a subsequent culture container by a tube are conducted repeatedly, whereby all culture containers are connected to the three-way stopcock, and
the culture medium is transferred from the culture medium supply container to each culture container in the multiple culture containers via the three-way stopcock by the pump.

2. The method of transferring a liquid between multiple containers in cell culture according to claim 1, wherein
the multiple culture containers comprise the first culture container and the second culture container,
the first culture container, a three-way stopcock and the culture medium supply container are connected by the tube, and one pump is arranged between the three-way stopcock and the culture medium supply container,
the three-way stopcock and the second culture container are connected by the tube, and
the culture medium is transferred from the culture medium supply container to the first culture container and the second culture container via the three-way stopcock by the pump.

3. The method of transferring a liquid between the multiple containers in cell culture according to claim 1, wherein
the multiple culture containers comprise the first culture container, the second culture container and a third culture container,
the first culture container, the three-way stopcock and the culture medium supply container are connected by the tube, and one pump is arranged between the three-way stopcock and the culture medium supply container,
the three-way stopcock and another three-way stopcock are connected by the tube, and the second culture container, another three-way stopcock and the third culture container connected by the tube, and
the culture medium is transferred from the culture medium supply container to the first culture container, the second culture container and the third culture container via the three-way stopcock by the pump.

4. The method of transferring a liquid between the multiple containers in cell culture according to any one of claims 1 to 3, wherein
each culture container in the multiple culture containers is arranged such that the horizontal position of the previously connected culture container becomes higher than the horizontal position of the subsequently connected culture container, and
transfer of a liquid from the previously connected culture container to the subsequently connected culture container is conducted by natural liquid transfer based on difference in horizontal position.

5. The method of transferring a liquid in cell culture according to any one of claims 1 to 4, wherein
a supernatant of a culture solution or a culture solution is transferred from the last one of the subsequently connected culture containers to the culture medium supply container via the three-way stopcock by a pump.

6. The method of transferring a liquid between multiple containers according to any one of claims 1 to 5, wherein
a three-way stopcock is provided on the tube between the culture medium supply container and the pump;
one or two or more another culture medium supply containers each having one port are connected to the culture medium supply container via a three-way stopcock by a tube; and
the culture medium is transferred from these culture medium supply containers to each culture container in the multiple culture containers via a three-way stopcock by the pump.
